(19) Europäisches Patentamt
European Patent Office
Office européen des brevets

(11) EP 3 178 891 B1

(12) **EUROPEAN PATENT SPECIFICATION**

(45) Date of publication and mention of the grant of the patent:
**21.10.2020 Bulletin 2020/43**

(21) Application number: **16202342.8**

(22) Date of filing: **16.03.2012**

(51) Int Cl.:
***C09D 171/02*** *(2006.01)*
***C08G 65/332*** *(2006.01)*
***C09D 163/00*** *(2006.01)*
***C07C 69/716*** *(2006.01)*
***C07C 69/78*** *(2006.01)*

# (54) LOW/ZERO VOC GLYCOL ETHER-ESTERS AS COALESCENTS FOR AQUEOUS POLYMERIC DISPERSIONS

VOC-ARME/-LOSE GLYKOLETHERESTER ALS KOALESZENZMITTEL FÜR WÄSSRIGE POLYMERDISPERSIONEN

ÉTHERS-ESTERS AU GLYCOL À TENEUR NULLE OU FAIBLE EN COV EN TANT QU'AGENTS DE COALESCENCE DE DISPERSIONS POLYMÈRES AQUEUSES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **08.04.2011 US 201161473243 P**
**01.07.2011 US 201161503647 P**

(43) Date of publication of application:
**14.06.2017 Bulletin 2017/24**

(62) Document number(s) of the earlier application(s) in accordance with Art. 76 EPC:
**12159798.3 / 2 508 577**

(73) Proprietors:
- **ROHM AND HAAS COMPANY**
**Philadelphia, PA 19106-2399 (US)**
- **Dow Global Technologies LLC**
**Midland, MI 48674 (US)**

(72) Inventors:
- **ADAMSON, Linda A.**
**Mt. Laurel, NJ New Jersey 08054 (US)**
- **ITTNER, Sarah E.**
**Saginaw, MI Michigan 48638 (US)**
- **BECKER, Michael C.**
**Dickinson, TX Texas 77539 (US)**
- **TEPE, Thomas R.**
**King of Prussia, PA Pennsylvania 19406 (US)**
- **DONATE, Felipe A.**
**Midland, MI Michigan 48642-6806 (US)**
- **WACHOWICZ, Rebecca J.**
**Bay City, MI Michigan 48706 (US)**
- **FASANO, David Michael**
**Maple Glen, PA Pennsylvania 19002 (US)**

(74) Representative: **Houghton, Mark Phillip et al**
**Patent Outsourcing Limited**
**1 King Street**
**Bakewell, Derbyshire DE45 1DZ (GB)**

(56) References cited:
**US-A1- 2005 228 092**
**US-A1- 2007 043 159**
**US-A1- 2009 198 002**
**US-A1- 2010 203 003**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

This invention relates to low and zero VOC glycol ether-ester compositions suitable for use as coalescents for aqueous polymeric dispersions. This invention particularly relates to glycol ether-ester coalescents of Formula (II)

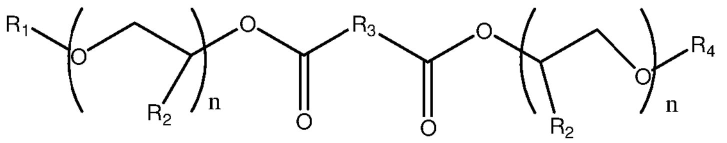

(II)

wherein $R_1$ and $R_4$ are, independently, $C_1$- $C_{10}$ alkyl groups, phenyl or benzyl, $R_2$ is either hydrogen or methyl, $R_3$ is a carbon chain including 1-2 carbon atoms, and n = 1 - 4; having a boiling point of greater than 450°C at 760 mm Hg, compositions including an aqueous polymeric dispersion and the low and zero VOC coalescents of the invention, and a method for forming a coating.

Coalescents are typically added to compositions such as, for example, aqueous polymeric dispersions and waterborne paints or coatings including aqueous dispersions of polymers to facilitate the formation of a continuous polymeric, or binder, film as water evaporates from the composition. Without the addition of coalescents polymer dispersions may not act as effective binders for pigments in the paint and adhesion to a substrate may be compromised. For many years, these coalescing aids have been relatively volatile solvents such as 2,2,4-trimethyl-1,3-pentanediol monoisobutyrate.

Volatile organic compound (VOC) emissions contribute to the creation of ozone, a main constituent of smog. In the US, VOC regulations established by the US Environmental Protection Agency (EPA) and enforced at the state level dictate the maximum concentration of volatile solvents in paints, clean up solvents, and other products. In Europe, VOC limits are defined by the 2004/42/EC Solvents Directive for Decorative Paints. VOC regulations have become more and more stringent and have affected the use of available coalescents.

The present invention serves to provide certain glycol ether-esters and low or zero VOC compositions including glycol ether-esters that are particularly suitable for use in compositions that include aqueous polymeric dispersions such as, for example, decorative and protective coatings for various substrates.

U.S. Patent No. 4,489,188 discloses coating compositions including aqueous latex polymers and 5 to 50 parts by weight amount of certain ether-ester solvents per 100 parts of polymer. Glycol ether-ester coalescents of the present invention are not disclosed.

U.S. Patent Application Publication No. 20090198002A1 discloses coalescent compositions for aqueous coating compositions including blends of dibasic esters such as bis-glycol ether esters of $C_4$ - $C_6$ diacids specifically, succinic, glutaric, and adipic acids, with maximum boiling points up to 450°C. Glycol ether-ester coalescents of the present invention are not disclosed.

There continues to be a need for low and no VOC coalescents for aqueous polymeric dispersions.

There is provided a glycol ether-ester coalescent selected from the group of compositions of Formula (II)

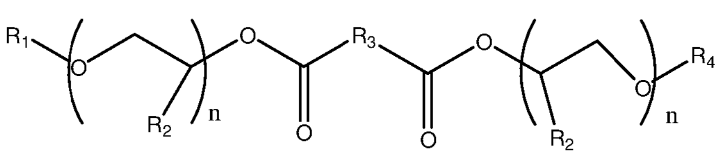

(II)

wherein $R_1$ and $R_4$ are, independently, $C_1$ - $C_{10}$ alkyl groups, phenyl or benzyl, $R_2$ is either hydrogen or methyl, $R_3$ is a carbon chain comprising 3-4 carbon atoms, and n = 1 - 4; and mixtures thereof; wherein the boiling point of said coalescent is greater than 450°C at 760 mm Hg.

There is provided an aqueous coating composition comprising an aqueous polymeric dispersion and from 0.1% to 40% by weight, based on the weight of aqueous polymeric dispersion solids, said glycol ether-ester coalescent of the present invention.

There is provided a method for forming a coating comprising (a) forming said aqueous coating composition of the fourth aspect of the present invention; (b) applying said aqueous coating composition to a substrate; and (c) drying, or allowing to dry, said applied aqueous coating composition.

The invention relates to a glycol ether-ester coalescent selected from the group of compositions of Formula (II)

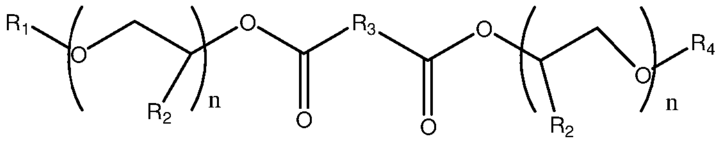

(II)

wherein $R_1$ and $R_4$ are, independently, $C_1$ - $C_{10}$ alkyl groups, phenyl or benzyl, $R_2$ is either hydrogen or methyl, $R_3$ is a carbon chain including 3-4 carbon atoms, and n = 1 - 4; and mixtures thereof; wherein the boiling point of said coalescent is greater than 450°C at 760 mm Hg.

In each instance herein R3 is a carbon chain including a certain number of carbon atoms; the chain may be, for example, saturated, unsaturated, substituted, part of a ring structure, or combinations thereof. The individual carbon atoms in the chain may bear substituent groups such as, for example, -OH, -Cl, =O, -NH2, and the like.

By "coalescent composition" is meant a composition that facilitates the film formation of an aqueous polymeric dispersion, particularly an aqueous coating composition that includes a dispersion of polymer in an aqueous medium such as, for example, a polymer prepared by emulsion polymerization techniques. An indication of facilitation of film formation is that the minimum film formation temperature ("MFFT") of the composition including the aqueous polymeric dispersion is measurably lowered by the addition of the coalescent.

The glycol ether-esters of the present invention are esters of monocarboxylic acids or dicarboxylic acids and glycol ethers, the latter obtained by reacting alcohols or phenol with either ethylene oxide or propylene oxide. Any of several synthetic methods known to those skilled in the art can be used to prepare the aforementioned esters. For instance, stoichiometric amounts of the glycol ether and the desired carboxylic acid can be heated in the presence of a catalytic amount of a strong acid such as, for example, concentrated sulfuric acid and p-toluene sulfonic acid and a solvent such as, for example, heptane, and water removed azeotropically to yield the desired product. Another method of preparation employs the acid monochloride (or dichloride) instead of the carboxylic acid as a reactant. In this case, hydrogen chloride gas is given off instead of water during the reaction of the acid chloride with the glycol ether. The hydrogen chloride may be trapped using a water scrubber. Still another method of preparation involves the transesterification of a simple alkyl ester of the desired acid with a glycol ether in the presence of a titanium catalyst such as tetraisopropyl titanate. Still another method of esterification uses the acid anhydride as reactant in combination with the azeotropic removal of water. This method is aimed at producing diesters. Glycol ether esters obtained by any of the aforementioned methods can be purified by flash distillation under high vacuum.

The structural requirements of the glycol ether esters of the clean-up solvent and paint thinner for solvent-borne resins and coatings of the invention have been set forth in Formula II. The glycol ether esters are typically liquids in the 0 - 25 °C temperature range to facilitate their use as thinners and clean up solvents. These products are desirably less than 10% volatile by Method 24, preferably less than 5% volatile, and most preferably less than 1% volatile to be useful as low VOC coalescing aids in the U.S. To be classified as VOC-exempt in the EU, the solvents must boil above 250°C and preferably above 280°C.

Bis-glycol ether esters described by Formula 2 were prepared from malonic acid, succinic acid, and maleic anhydride. Glycol ethers used in these preparations were ethylene glycol n-hexyl ether, triethylene glycol n-hexyl ether, dipropylene glycol 2-ethylhexyl ether, diethylene glycol n-hexyl ether, diethylene glycol phenyl ether, diethylene glycol n-butyl ether, dipropylene glycol phenyl ether, tripropylene glycol n-pentyl ether, dipropylene glycol methyl ether, tripropylene glycol methyl ether, dipropylene glycol n-butyl ether, dipropylene glycol n-propyl ether, tripropylene glycol n-propyl ether, propylene glycol n-butyl ether, tripropylene glycol n-butyl ether, triethylene glycol n-butyl ether, propylene glycol methyl ether, triethylene glycol n-pentyl ether, and ethylene glycol n-pentyl ether. Ethylene glycol phenyl ether and propylene glycol phenyl ether were used to prepare benzoates and succinates but the resulting glycol ether esters were solids melting in the 50 - 100 °C range which limits their utility as coalescents.

The aqueous coating composition of the present invention includes an aqueous polymeric dispersion and from 0.1% to 40% by weight, based on the weight of aqueous polymeric dispersion solids, of the coalescent of the present invention. In one embodiment when the MFFT of the aqueous polymeric dispersion is from -5°C to 100°C, from 0.1% to 30% coalescent, by weight based on the weight of aqueous polymeric dispersion solids, may be used. Alternatively, when the MFFT of the aqueous polymeric dispersion is from -20°C to 30°C, from 0.1% to 5% coalescent, by weight based on the weight of aqueous polymeric dispersion solids, may be used. MFFTs of the aqueous polymeric dispersions herein are those measured using ASTM D 2354 and a 5 mil MFFT bar. MFFT values are indicative of how efficient a coalescent is for a given aqueous polymeric dispersion; it is desirable to achieve the lowest possible MFFT with the smallest amount of coalescent. The aqueous polymeric dispersion may be a dispersion of a polymer, oligomer, or prepolymer in an aqueous medium. In some embodiments the aqueous polymeric dispersion may be reactive before, during, or subsequent to film formation. By "aqueous medium" is meant herein a medium including at least 50%, by weight based on the weight of the medium, water. Typical aqueous polymeric dispersions are aqueous dispersions of epoxies, urethanes, acrylic polyols, polyesters, and hybrids of these and other chemistries; and emulsion polymers.

In some embodiments the aqueous polymeric dispersions are part of reactive systems. For example, in a 2k system such as an epoxy dispersion system the coalescent can be added to either the component including the epoxy dispersion or, alternatively to the curing agent component or split between both components of the system.

The emulsion polymer, an aqueous dispersion of polymer formed by emulsion polymerization techniques, includes at least one addition copolymerized ethylenically unsaturated monomer such as, for example, styrene or substituted styrenes; vinyl toluene; butadiene; (meth)acrylonitrile; a (meth)acrylic ester monomer such as, for example, methyl (meth)acrylate, ethyl (meth)acrylate, butyl (meth)acrylate, hydroxyethyl (meth)acrylate, hydroxypropyl (meth)acrylate, and ureido-functional (meth)acrylates; vinyl acetate or other vinyl esters; vinyl monomers such as vinyl chloride, vinylidene chloride, and N-vinyl pyrollidone. The use of the term "(meth)" followed by another term such as (meth)acrylate, as used throughout the disclosure, refers to both acrylates and methacrylates.

In certain embodiments the emulsion polymer includes from 0% to 6%, or in the alternative, from 0% to 3 wt% or from 0% to 1%, by weight based on the weight of the polymer, of a copolymerized multi-ethylenically unsaturated monomer. It is important to select the level of multi-ethylenically unsaturated monomer so as to not materially interfere with film formation and integrity. Multi-ethylenically unsaturated monomers include, for example, allyl (meth)acrylate, diallyl phthalate, 1,4-butylene glycol di(meth)acrylate, 1,2-ethylene glycol di(meth)acrylate, 1,6-hexanediol di(meth)acrylate, and divinyl benzene.

The emulsion polymer includes from 0% to 15%, preferably from 0.5% to 5%, of a copolymerized monoethylenically-unsaturated acid monomer, based on the weight of the polymer. Acid monomers include carboxylic acid monomers such as, for example, (meth)acrylic acid, crotonic acid, itaconic acid, fumaric acid, maleic acid, monomethyl itaconate, monomethyl fumarate, monobutyl fumarate, maleic anhydride, 2-acrylamido-2-methylpropane sulfonic acid, vinyl sulfonic acid, styrene sulfonic acid, 1-allyloxy-2-hydroxypropane sulfonic acid, alkyl allyl sulfosuccinic acid, sulfoethyl (meth)acrylate, phosphoalkyl (meth)acrylates such as phosphoethyl (meth)acrylate, phosphopropyl (meth)acrylate, and phosphobutyl (meth)acrylate, phosphoalkyl crotonates, phosphoalkyl maleates, phosphoalkyl fumarates, phosphodialkyl (meth)acrylates, phosphodialkyl crotonates, and allyl phosphate.

The aqueous emulsion polymer is typically formed by an addition polymerization emulsion polymerization process as is known in the art. Conventional surfactants and blends may be used including, for example, anionic and/or nonionic emulsifiers such as, for example, alkali metal or ammonium alkyl sulfates, alkyl sulfonic acids, fatty acids, and oxyethylated alkyl phenols, and mixtures thereof. Polymerizable surfactants that include at least one ethylenically unsaturated carbon-carbon bond which can undergo free radical addition polymerization may be used. The amount of surfactant used is usually 0.1% to 6% by weight, based on the weight of total monomer. Either thermal or redox initiation processes may be used. Conventional free radical initiators may be used such as, for example, hydrogen peroxide, t-butyl hydroperoxide, t-amyl hydroperoxide, ammonium and/or alkali persulfates, typically at a level of 0.01% to 3.0% by weight, based on the weight of total monomer. Redox systems using the same initiators coupled with a suitable reductant such as, for example, sodium sulfoxylate formaldehyde, sodium hydrosulfite, isoascorbic acid, hydroxylamine sulfate and sodium bisulfite may be used at similar levels, optionally in combination with metal ions such as, for example iron and copper, optionally further including complexing agents for the metal. Chain transfer agents such as mercaptans may be used to lower the molecular weight of the polymer. The monomer mixture may be added neat or as an emulsion in water. The monomer mixture may be added in a single addition or more additions or continuously over the reaction period using a uniform or varying composition. Additional ingredients such as, for example, free radical initiators, oxidants, reducing agents, chain transfer agents, neutralizers, surfactants, and dispersants may be added prior to, during, or subsequent to the monomer addition. Processes yielding polymodal particle size distributions such as those disclosed in US Patent Nos. 4,384,056 and 4,539,361, for example, may be employed. The emulsion polymer may be formed in a multi-stage emulsion polymerization process as are well known in the art. The emulsion polymer is also contemplated to be formed in two or more stages, the stages differing in molecular weight. Blending two different emulsion polymers is also contemplated.

The average particle diameter of the emulsion polymer particles is typically from 40 nm to 1000 nm, preferably from 40 nm to 300 nm. Particle diameters herein are those measured by dynamic light scattering on a Brookhaven BI-90 Plus particle size analyzer.

The aqueous coating composition of the invention is prepared by techniques which are well known in the coatings art. First, pigment(s), if any, are well dispersed in an aqueous medium under high shear such as is afforded by a COWLES™ mixer or predispersed colorant(s), or mixtures thereof are used. Then the emulsion polymer is added under low shear stirring along with the coalescent composition and other coatings adjuvants as desired. The aqueous coating composition may include, in addition to the aqueous polymeric dispersion and optional pigment(s), conventional coatings adjuvants such as, for example, extenders, emulsifiers, coalescing agents other than the coalescent composition of the present invention, plasticizers, antifreezes, curing agents, buffers, neutralizers, thickeners, rheology modifiers, humectants, wetting agents, biocides, plasticizers, antifoaming agents, UV absorbers, fluorescent brighteners, light or heat stabilizers, biocides, chelating agents, dispersants, colorants, waxes, and water-repellants.

Examples of suitable pigments and extenders include titanium dioxide such as anatase and rutile titanium

dioxides; zinc oxide; antimony oxide; iron oxide; magnesium silicate; calcium carbonate; organic and inorganic colored pigments; aluminosilcates; silica; various clays such as kaolin and delaminated clay; and lead oxide. It is also contemplated that the aqueous coating composition may also contain opaque polymer particles, such as, for example, Ropaque™ Opaque Polymers (Dow Chemical Co.). Also contemplated are encapsulated or partially encapsulated opacifying pigment particles; and polymers or polymer emulsions adsorbing or bonding to the surface of pigments such as titanium dioxide; and hollow pigments, including pigments having one or more voids.

Titanium dioxide is the main pigment used to achieve hiding in architectural paints. This pigment is expensive and in short supply. One way to achieve hiding while decreasing the amount of $TiO_2$ is to include multistage emulsion polymers that add opacity to the paint film, commonly known as "opaque polymers". These polymers are water-filled emulsion polymer particles (mostly styrene) with a high Tg. These particles fill with air during film formation and scatter light creating opacity. Typically an aqueous coating composition including an opaque polymer will also include an aqueous polymeric dispersion; desirably a coalescent will facilitate film formation of the aqueous polymeric dispersion, but not cause the opaque polymer to collapse. However, some coalescents attack the opaque polymer causing the particles to collapse which results in less light scattering and decreased opacity. TEXANOL™, for example, attacks the opaque polymers when used at 15% by weight on resin solids while the low VOC plasticizer OPTIFILM™ 400 attacks the polymer at much lower levels (about 6% by weight on resin solids). Certain glycol ether-ester and diester coalescents of the invention were useful in their ability to preserve the opacity provided by certain commercial ROPAQUE™ opaque polymers. Preferred are dipropylene glycol phenyl ether benzoate (DiPPh Benzoate), bis-dipropylene glycol n-butyl ether adipate (DPnB Adipate), bis-dipropylene glycol n-propyl ether adipate (DPnP Adipate), bis-dipropylene glycol n-butyl ether maleate (DPnB Maleate), and tripropylene glycol pentyl ether benzoate (TPP Benzoate).

The amounts of pigment and extender in the aqueous coating composition vary from a pigment volume concentration (PVC) of 0 to 85 and thereby encompass coatings otherwise described in the art, for example, as clear coatings, stains, flat coatings, satin coatings, semi-gloss coatings, gloss coatings, primers, textured coatings, and the like. The aqueous coating composition herein expressly includes architectural, maintenance, and industrial coatings, caulks, sealants, and adhesives. The pigment volume concentration is calculated by the following formula:

$$\text{PVC (\%)} = \frac{\text{volume of pigment(s), + volume extender(s) x 100.}}{\text{total dry volume of paint}}$$

The solids content of the aqueous coating composition may be from 10% to 70% by volume. The viscosity of the aqueous coating composition may be from 50 centipoises to 50,000 centipoises, as measured using a Brookfield viscometer; viscosities appropriate for different application methods vary considerably.

In the method for forming a coating of the invention the aqueous coating composition is typically applied to a substrate such as, for example, wood, metal, plastics, marine and civil engineering substrates, cementitious substrates such as, for example, concrete, stucco, and mortar, previously painted or primed surfaces, and weathered surfaces. The aqueous coating composition may be applied to a substrate using conventional coatings application methods such as, for example, brush, roller, caulking applicator, roll coating, gravure roll, curtain coater and spraying methods such as, for example, air-atomized spray, air-assisted spray, airless spray, high volume low pressure spray, and air-assisted airless spray.

Drying of the aqueous coating composition to provide a coating may be allowed to proceed under ambient conditions such as, for example, at 5 °C to 35°C. or the coating may be dried at elevated temperatures such as, for example, from 35 °C to 150°C.

The invention in some of its embodiments will now be further described by reference to the following examples:

Test methods:

Methods used in Example 5

All of the tests below have the same sample prep steps as defined:
10mil wet film thickness draw down on Bonderite 1000 treated steel panels (except for Early Water Resistance, which was done on untreated aluminum.

Drying/curing time was 7 days for chemical resistance, impact resistance and mandrel bend flexibility.

Drying/curing time for Konig and Pencil hardnesses are as given in the data table (test was done at numerous cure times).

Early Water Resistance (EWR) was performed on separate panels that were dried for 4 or 6 hours, as noted in the data table.

Test methods that are ASTM:

Dry to Touch and Handle = ASTM D1640

Chemical Resistance = Spot test in ASTM D 1308. Chemicals are as listed in the table and were allowed to remain in contact for either 24 or 48 hrs as noted in the data table.

Konig Hardness = ANS/ISO 1522 (formerly ASTM D4366)

Pencil Hardness = ASTM D3363

Impact Resistance = ASTM D2794

Mandrel Flex = ASTM D522

EWR Test method- After drawdown, allowed panel to dry for prescribed time (4 or 6 hours) at 77F/50%RH- Panels were then placed in fog box for at least 18 hours, then removed, wiped dry and immediately rated for degree of blistering, as per ASTMD714.

Methods used in Example 6:

LTFF (low temperature film formation) at 40°F/40% RH: This is a procedure for determining the ability of a paint film to form a continuous film at low temperatures. Aqueous coating compositions were drawdown in a room conditioned at 40°F/40% RH with a 10 mil block bar over a Leneta B&W sealed and unsealed chart. Paints were dried for 2 4 hours and then rated for cracking, 1 day Gloss: Paints were drawdown in CTR (constant temperature room conditions) with a 3 mil bird. After 12 hours, 20 and 60 degree gloss was measured Brushed flow: test paints were brushed out by natural spread rate on a Spreading Rate chart and allowed to dry. Flow was rated on a scale of 1-10, with 10 being the best.

Contrast Ratio (C) - Contrast ratio is the ratio of the reflectance of a dry paint film over a black substrate of 2% or less reflectance to the reflectance of the same paint, equivalently applied and dried, over a substrate of 80% reflectance (ASTM D-2805.88). C is a function of film thickness and toner concentration

Color Acceptance: This is a measure of how well a colorant is accepted by the tint-base. Good color acceptance is required in order to match color chips and give uniform color appearance. Color acceptance of paints is necessary under various conditions of paint age and shear during application. We added 4 ounces/gallon of Phthalo Blue predispersed colorant to the tint base paint then followed this procedure:

1. A film was drawn down with a 3 mil Bird film applicator on a 1B Penopac chart held by a vacuum plate.
2. Two small sections approximately 1-2 inches in diameter (one in the sealed area and one in the unsealed area) were rubbed in a circular motion with clean, dry finger tips.

    ◦ The area rubbed on the unsealed, bottom third was rubbed until almost dry or 100 cycles, so a high degree of shear was generated.
    ◦ The area rubbed on the sealed, middle third was rubbed for approximately 100 cycles and represents a low shear state.

3. The charts were dried in the CTR 24 hours before rating
4. Rating scale: no change to various degrees of possible colorant (dark) or TiO2 flocculation (light)

Yellowing: After heat aging the aqueous coating composition for 10 days at 140°F (60°C), the composition was equilibrated for 24 hours then drawdown on a white chart side-by-side with its non-heat aged retain. After drying overnight, any changes in color with the heat aged paint system were recorded.

Abrasive Scrub Resistance: This test measures the scrub resistance of a paint film by the number of cycles required to erode the paint film to the substrate. Cut through indicates an area of film removed that is the width of the drawdown of the original dried film. Aqueous coating compositions were drawdown on a black vinyl chart, allowed to dry 5-7 days, and then scrubbed using a Gardner Abrasive Scrub machine. To accelerate the failure, a plate with a brass shim (Shim, 10 mils x ½" x 6-1/2) was used

Stain Removal: This test method describes the procedure for evaluating the ease of removing common household stains from a paint film with a non-abrasive detergent. Aqueous coating compositions were drawdown on a black vinyl chart and allowed to dry for 5-7 days. Common household stains were applied to the film and allowed to dry for 60

minutes before being placed on a Gardner Scrub Tester and "washed" with cheesecloth that was saturated with 1% Tide solution for 200 scrub cycles. Stains include:
Hydrophobic: lipstick, #2 pencil, ballpoint pen, crayon, red sharpie marker, red china marker.

Hydrophilic: tea, mustard, grape juice, coffee, ketchup or spaghetti sauce, red wine, black flair.

1 day Hot Block: This test measures the tendency of painted surfaces to stick together (block) when stacked or placed in contact with each other under pressure. Tack is the noise produced upon separation of blocked surfaces; Seal is the physical damage to a paint film caused by the separation. The procedure follows:

1. The aqueous coating composition to be tested was drawn down on a chart using a 3 Mil Bird applicator. Panels were conditioned in the CTR (25°C; 50% RH) for 7 days.
2. Weights and stoppers were equilibrated in the oven overnight prior to running the test. Cut out four 1-1/2" x 1-1/2" sections (to run duplicates) from white area of each conditioned panel.
3. The cut sections were positioned with the paint surfaces face to face.
4. The face to face specimen was placed in a 50°C (120°F) oven on a flat metal plate. Each individual specimen was topped with a heated, solid, number 8 rubber stopper with narrow side down and a heated 1000 g. weight placed on each stopper. The force calculated for this setup is 127 g/cm2 (1.8 psi).
5. After exactly 30 minutes ($\pm$1 min.), the stoppers and weights were removed and the test sections removed from the oven. The test specimens were allowed to cool for 30 minutes at room temperature.
6. After cooling, the sections were separated with slow and steady force. They were pulled apart at an angle of approximately 180° while listening for tack. The samples were rated for block resistance on a scale of 0 to 10.

Print Resistance: This test measures the ability of a coating to resist the imprint of another surface placed on it. The procedure follows:

1. The aqueous coating composition to be tested was cast on an aluminum panel using a drawdown block with 5 mil opening. The coated aluminum panels were conditioned in the CTR (77°F, 50% R.H.) for 1, 3, and 7 days.
2. After the panels had been conditioned 1, 3, or 7 days, approximately 1-1/2" x 1-1/2" of aluminum panels were cut out, 1-1/2" pieces of cheesecloth, two pieces for each test panel. (Note: cheesecloth was used as supplied with all 4 layers intact.)
3. Weights and stoppers were put in the 140°F oven to equilibrate (day before).
4. One piece of cheesecloth (one at top, one at bottom) was placed over each test specimen and topped with a number 8 stopper and a 500 gram weight, using one weight and stopper for each area to be tested in the oven for 60 minutes.
5. After 60 minutes, stoppers and weights were removed and test specimens removed from the oven. Specimens were allowed to cool (about ½ hour) before removing the cheesecloth and evaluating for print.
6. Cheesecloth was removed and the paint film under the cheesecloth carefully examined. The depth and the amount of the impression of the cheesecloth pattern which was left imprinted on the paint film surface was rated on a scale of 0 to 10.

Konig Hardness: The Byk Mallinckrodt Konig Pendulum Hardness Tester measures how hard a film is by the use of a pendulum. The harder the film surface, the more time the pendulum will swing and, thus, the higher the recorded count. The softer the film, the more friction the pendulum will experience and will therefore swing freely fewer times. This will result in a lower recorder count.

Lab DPUR (Dirt Pick Up Resistance): This test measures the ability of a paint film to resist the deposit of foreign matter consisting of dirt, soot, or stain present on the surface of exposed exterior coated panels. This test method provides for visual comparison, as well as Y reflectance readings before and after exposure, and the difference is considered to be dirt collection. The procedure follows:

1. The test aqueous coating compositions were drawn down with a 5 mil Block bar on an Aluminum panel and let dry overnight.
2. The test panels were exposed for 5-7 days outside (S-45 direction preferred). The panels were brought in and allowed to air dry
3. Applied by brush to a ¼ of the test paint Mapico 422 brown iron oxide slurry. Allowed the slurry to completely dry (minimum 4 hours).
4. Washed the slurry off under water using a clean piece of cheesecloth and gentle, consistent pressure.
5. Allowed panels to dry. Took reflectance readings of both the untreated and treated areas. The higher the number, the better the DPUR

EXAMPLE 1 - Preparation of Glycol Ether Esters using Maleic Anhydride

Reactions were conducted in a 250-ml one-neck flask equipped with a magnetic stirrer, a heating mantle, a built-in thermocouple well, a heating mantle connected to a temperature controller fitted with control and high limit thermocouples, and a Dean-Stark trap connected to a condenser bearing a nitrogen adapter teed-off to a bubbler. The glycol ether, maleic anhydride, heptane solvent, and the sulfuric acid catalyst were placed in the flask. The apparatus was placed under a nitrogen blanket and the contents heated to about 60°C to get the maleic anhydride to melt and react with the glycol ether. After the initial ring-opening reaction and subsequent exotherm, the reaction mixture was heated to about 118 °C to establish a constant heptane reflux through the trap where the water of esterification was collected. The reaction was allowed to continue until the theoretical volume of water was collected. A typical Example 1synthesis follows:
Into the flask were placed 106.72g (0.56 mole) dipropylene glycol n-butyl ether, 25.11g (0.26mole) maleic anhydride, 60 ml heptane, and 4 drops concentrated sulfuric acid. The reactor was placed under a nitrogen blanket. The contents were heated to about 60 °C to get the maleic anhydride to melt and react with the glycol ether. After observing the exotherm, the reaction mixture was heated to about 118 °C to establish a constant heptane reflux. The reaction was allowed to continue for a total of 15 hours, at which point most of the theoretical amount of water was collected. The reaction mixture was cooled to 25 °C and analyzed neat by gas chromatography on a 30m x 0.25mmID x 0.25 micron film ZB-5 capillary column from Phenomenex. The area percent GC chromatogram showed about 16% residual glycol ether and about 83% of a product tentatively identified as bis-dipropylene glycol n-butyl ether maleate (solvent was excluded from the chromatogram area summary). The reaction mixture was then filtered through a small bed of activated basic alumina to neutralize the catalyst. The filtrate was placed in a boiling flask and the heptane removed at low pressure in a Büchi rotary evaporator. The residue was flash distilled under vacuum to isolate the product in 99.2% purity boiling at 195 °C @ 0.1 mmHg. The product was confirmed as bis-dipropylene glycol n-butyl ether maleate by its IR and NMR spectra The boiling point at reduced pressure was corrected to the normal boiling by means of a computer program that fits vapor pressure data to an Antoine equation of the form $\log P = A - B/(T + C)$. The normal boiling point was calculated as 476 °C. A sample of the product was then tested as specified by EPA Method 24 and found to contain only 0.1 percent volatiles. Another sample of the product was subsequently evaluated in the standard MFFT test as a coalescing agent for an acrylic emulsion polymer (RHOPLEX™ SG-30) at a concentration of 5% by weight based on resin solids. The MFFT value obtained was 41 °F, a value 24% lower than the MFFT obtained for the neat latex and 6% lower than the MFFT obtained with TEXANOL™. (See Table 1.2)

EXAMPLE 2. Preparation of Glycol Ether Esters using Acid Chlorides

Reactions were conducted in a 250-mL three-necked round-bottom flask equipped with an addition funnel with pressure equalizing side-arm, a cooling condenser, a thermocouple well, a magnetic Teflon stirring bar, and a heating mantle connected to a temperature controller fitted with control and high limit thermocouples. The addition funnel was equipped with an adapter connected to a low pressure nitrogen line. The condenser was fitted with an adapter connected to a glass trap filled with water. In a typical reaction, the glycol ether was loaded into the flask and a stoichiometric amount of the acid chloride added slowly to control the exotherm and the HCl generation. Reactions were followed by gas chromatography and the products verified by their IR and NMR spectra. A typical Example 2 synthesis follows:
Into the reaction flask was loaded 50.0 g (0.28 mole) diethylene glycol phenyl ether. Benzoyl chloride (31.8 ml, 38.58g, 0.28 mole) was added into the addition funnel, the nitrogen adapter was placed on the funnel, and a slow nitrogen flow was started as evidenced by the bubbling in the water trap. A magnetic stirrer was placed beneath the mantle to start the agitation. The benzoyl chloride was added dropwise over a one-hour period during which the temperature was allowed to rise to about 80 °C. Hydrogen chloride gas emitted was captured in the trap. Once the addition was complete, the temperature was adjusted to about 112 °C and maintained there for two hours. The reaction mixture was then allowed to cool to room temperature so that a small sample could be withdrawn with a syringe. The sample was diluted with isopropanol containing tetradecane as an internal standard and analyzed by gas chromatography on a 30m x 0.25mmID x 0.25 micron film RTX200 capillary column from Restek. The analysis showed that the reaction mixture contained 0.28% residual diethylene glycol phenyl ether and 95.5% of a major component tentatively identified as the diethylene glycol phenyl ether benzoate. The reaction mixture was flash-distilled under reduced pressure to recover 72.6g product with 99.1% purity boiling at 180 °C @ 0.5 mmHg. The product was confirmed as diethylene glycol phenyl ether benzoate by its IR and NMR spectra. The boiling point obtained under reduced pressure was fitted to the Antoine equation of the form $\log P = A - B/(T + C)$ constrained using Thompson's rule and a Trouton constant of 22 to obtain a normal boiling point of about 440 °C. The product was evaluated in the MFFT test and by EPA Method 24 as described in Example 1. (Results in Table 1.2)

EXAMPLE 3. Preparation of Glycol Ether Levulinates by Transesterification

Several glycol ether levulinates were prepared by transesterification of ethyl levulinate. In these syntheses, the glycol ether was placed in a 100-ml, 3-necked, round-bottom flask equipped with a built-in thermocouple well, a 50-ml addition funnel with pressure equalizing arm fitted with a nitrogen adapter, a distillation head with condenser, vacuum/nitrogen adapter, and a 25-ml graduated receiver, a Teflon stirring bar, a glass stopper, and a heating mantle connected to a temperature controller fitted with control and high limit thermocouples. The distillation head was connected to a nitrogen bubbler through the nitrogen adapter. The entire apparatus was secured on top of a magnetic stir plate. The apparatus was swept with nitrogen from the addition funnel to the bubbler. The titanium tetraisopropoxide transesterification catalyst (DuPont's Tyzor® TPT) was added and the mixture heated to activate the catalyst. An equimolar amount of ethyl levulinate was then added slowly and the ethanol collected in the receiver. Eventually the nitrogen purge was replaced with a vacuum pull after cooling the receiver with dry ice. The ethanol removed was monitored throughout the reaction. A typical Example 3 synthesis follows:
After purging the apparatus with nitrogen, 42.6g (0.17 moles) tripropylene glycol n-butyl ether was added to the reaction flask. About 1 ml of the titanium tetraisopropoxide catalyst was loaded into a syringe (inside a nitrogen box) and then added to the glycol ether in the flask by momentarily lifting the glass stopper. The mixture was heated to 150°C. At this point, 27.8g (0.19 moles) ethyl levulinate was loaded into the addition funnel. Once the reaction mixture had stabilized at 150°C, the ester was added dropwise over a 40-60 minute period. As ethanol formed, it was collected in the receiver and the temperature gradually increased to 175°C. About one-half of the theoretical alcohol was removed during the first 3 hours. At this point, the nitrogen purge and the addition funnel were replaced with a glass stopper and the connection to the bubbler replaced with a vacuum line. The receiver was replaced with a small cold trap surrounded with dry ice. The pressure was slowly lowered to about 40mmHg (from either a water aspirator or vacuum pump) over the course of 4 to 5 hours. At that point, the reaction was discontinued. The total ethanol collected was recorded and the reaction product sampled and analyzed neat by gas chromatography on a 30m x 0.25mmID x 0.25 micron film ZB-5 capillary column from Phenomenex. The area percent GC chromatogram showed the presence of residual ethanol, unknown components, about 20% unreacted glycol ether, and about 61% of the product tentatively identified as tripropylene glycol n-butyl ether levulinate.

The catalyst was neutralized with ~0.25g deionized water and about 50 ml methyl ethyl ketone (MEK) was added to the flask while stirring the reaction mixture. A 12-inch x 1-inch ID glass column fitted with a fritted bottom and Teflon stopcock was filled with approximately 3 inches of neutral alumina. The reaction mixture was added slowly to the column and then a slight nitrogen pressure was applied on top of the column through an adapter to speed up the flow of material through the alumina. Additional MEK was added to recover any product clinging to the alumina. The MEK solution was then evaporated in a Büchi rotary evaporator with the water bath at 40°C and the pressure slowly reduced down to 0.2 mmHg until no more dripping was observed from the dry ice condenser. The residue was vacuum-distilled in a small flash distillation apparatus equipped with a cow fraction cutter. Fraction #2 weighed 24.0g and boiled at 150 - 160°C @ 0.4 mmHg. This product (94.5% pure) was positively identified as tripropylene glycol n-butyl ether levulinate by its IR and NMR spectra. The boiling point obtained under reduced pressure was fitted to the Antoine equation of the form $\log P = A - B/(T + C)$ constrained using Thompson's rule and a Trouton constant of 22 to obtain a normal boiling point of about 403°C. The product was evaluated in the MFFT test and by EPA Method 24 as described in Example 1. (Results in Table 1.2)

EXAMPLE 4. Preparation of Glycol Ether Esters by Direct Esterification with Carboxylic Acids

Glycol ether esters were prepared by direct esterification of the glycol ether with monocarboxylic or dicarboxylic acids in the presence of concentrated sulfuric acid and an azeotroping solvent such as, for example, heptane. In a typical reaction the glycol ether, the carboxylic acid, heptane, and the catalyst were loaded into a single neck flask equipped with a magnetic Teflon stirring bar, a built-in thermocouple well, and a heating mantle connected to a temperature controller fitted with control and high limit thermocouples. The flask was attached to a Dean-Stark trap itself connected to a reflux condenser bearing a nitrogen adapter teed-off to a bubbler. A magnetic stirrer plate was placed beneath the mantle. The entire apparatus was clamped to a fume hood lattice. After establishing a nitrogen blanket, the reaction mixture was stirred and heated to 120 - 130 °C to establish a constant heptane reflux through the trap where the water of esterification was collected. The reaction was allowed to continue until the theoretical volume of water was collected. A typical Example 4 synthesis follows:
Into a 2-L reaction flask were placed 298.1g (2.04 moles) adipic acid, 775.8g (4.08 moles) dipropylene glycol n-butyl ether, 352 ml heptane, and 1.35g (0.0138 moles) concentrated sulfuric acid. After establishing a nitrogen blanket and starting the stirrer, the reaction mixture was heated to 121°C to initiate a constant heptane reflux. After 5 hours, 45.7g water had been drained from the trap. The flask temperature was increased to 130°C and the reaction allowed to continue overnight for a total of about 30 hours at which point heating was discontinued. A total of 70.85g (3.94 moles) water was

collected (about 97% of theoretical). A sample of the reaction mixture was analyzed by gas chromatography on a 30m x 0.25mmID x 0.25 micron film ZB-5 capillary column from Phenomenex. The area percent GC chromatogram showed about 1.8% residual glycol ether and about 93.3% of a product tentatively identified as bis-dipropylene glycol n-butyl ether adipate (the solvent was excluded from the chromatogram area summary). The reaction mixture was then filtered through a small bed of activated basic alumina to neutralize the catalyst. The filtrate was placed in a boiling flask and the heptane removed at low pressure in a Büchi rotary evaporator. The residue was flash distilled under vacuum to isolate 806.4g product (80% yield) boiling at 204 - 211 °C @ 0.2 mmHg(95.5% purity). The product was confirmed as bis-dipropylene glycol n-butyl ether adipate by its IR and NMR spectra The boiling point at reduced pressure was corrected to the normal boiling by means of a computer program that fits vapor pressure data to an Antoine equation of the form logP = A - B/(T + C). The normal boiling point was calculated as 485 °C. The product was evaluated in the MFFT test and by EPA Method 24 as described in Example 1. (Results in Table 1.2)

Table 1.1 Synthesis of glycol ether-esters

| Glycol Ether | | Carboxylic acid or Ester Used | | Solvent used | Catalyst Used | Crude Rx Mixture Composition (Area %) | | Distilled Product | Preparation method |
|---|---|---|---|---|---|---|---|---|---|
| Abbreviation | Grams | Name | Grams | | (Grams or mL) | Glycol Ether | Product | Area% by GC | Method of Example |
| DiEPh | 32.00 | Ethyl Levulinate | 24.5 | none | 1 ml TYZOR™ TPT | 24.6 | 67.7 | 97.5 | 3 |
| HxCb | 33.40 | Ethyl Levulinate | 24.5 | none | 1 ml TYZOR™ TPT | 17.8 | 76.7 | 94.5 | 3 |
| HxCs | 26.40 | Ethyl Levulinate | 24.5 | none | 1 ml TYZOR™ TPT | 8.8 | 82.9 | 95.9 | 3 |
| DiPPh | 37.40 | Ethyl Levulinate | 24.5 | none | 1 ml TYZOR™ TPT | 27.9 | 66.6 | 94.3 | 3 |
| TPM | 35.50 | Ethyl Levulinate | 24.5 | none | 1ml TYZOR™ TPT | 13.9 | 73.8 | 96.7 | 3 |
| TPnP | 39.73 | Ethyl Levulinate | 26.2 | none | 1 ml TYZOR™ TPT | 20.1 | 69.9 | 95.3 | 3 |
| BTG | 36.60 | Ethyl Levulinate | 26.70 | none | 1 ml TYZOR™ TPT | 14.5 | 76.1 | 94.3 | 3 |
| TPnB | 42.60 | Ethyl Levulinate | 27.80 | none | 1 ml TYZOR™ TPT | 20.3 | 60.9 | 94.5 | 3 |
| DiEPh | 97.60 | Ethyl Levulinate | 82.40 | none | 1 ml TYZOR™ TPT | 18.0 | 50.8 | 94.6 | 3 |
| TPnB | 262.00 | Levulinic Acid | 121.50 | heptane | 1.02g $H_2SO_4$ | 6.2 | 85.6 | >97 | 4 |
| DiEPh | 198.10 | Levulinic Acid | 124.00 | heptane | 1.02g $H_2SO_4$ | 0.9 | 97.7 | >95 | 4 |
| BuCb | 45.70 | Adipic Acid | 20.20 | heptane | 0.11 g $H_2SO_4$ | 5.1 | 85.6 | 88.2 | 4 |
| DPnP | 77.10 | Adipic Acid | 31.80 | heptane | 0.2g $H_2SO_4$ | 11.7 | 77.1 | >98 | 4 |
| DPM | 74.60 | Adipic Acid | 35.90 | heptane | 0.2g $H_2SO_4$ | 5.2 | 70.0 | >99 | 4 |
| HxCs | 74.60 | Adipic Acid | 36.30 | heptane | 0.23g $H_2SO_4$ | 3.7 | 92.9 | >97 | 4 |
| PM | 186.21 | Adipic Acid | 50.59 | none | 0.27g $H_2SO_4$ | 2.0 | 96.9 | 99.0 | 4 |
| DPnB | 775.80 | Adipic Acid | 298.10 | heptane | 1.35g $H_2SO_4$ | 1.8 | 93.3 | 95.5 | 4 |

(continued)

| Glycol Ether | | Carboxylic acid or Ester Used | | Solvent used | Catalyst Used | Crude Rx Mixture Composition (Area %) | | Distilled Product | Preparation method |
|---|---|---|---|---|---|---|---|---|---|
| HxCs | 86.80 | Succinic Acid | 31.80 | heptane | 0.21 g $H_2SO_4$ | 5.4 | 90.5 | >97 | 4 |
| BuCb | 108.80 | Succinic Acid | 36.30 | heptane | 0.22g $H_2SO_4$ | 7.2 | 91.7 | >98 | 4 |
| DiEPh | 143.00 | Isopentanoic Acid | 51.00 | heptane | 0.25g $H_2SO_4$ | 23.4 | 75.6 | >98 | 4 |
| BTG | 103.70 | Isopentanoic Acid | 51.00 | heptane | 0.26g $H_2SO_4$ | 4.0 | 89.8 | >98 | 4 |
| HTG | 117.00 | Isopentanoic Acid | 51.00 | heptane | 0.26g $H_2SO_4$ | 1.9 | 96.2 | >98 | 4 |
| TPnB | 124.20 | Isopentanoic Acid | 51.00 | heptane | 0.2g $H_2SO_4$ | 8.7 | 86.9 | >98 | 4 |
| HTG | 117.00 | Valeric Acid | 51.00 | heptane | 0.2g $H_2SO_4$ | 0.9 | 96.1 | >99 | 4 |
| | | | | | | | | | |
| BTG | 103.60 | Acetyl Chloride | 47.00 | none | none | 2.1 | 85.9 | >86 | 2 |
| BuCs | 59.00 | Benzoic Acid | 61.00 | heptane | 0.12g $H_2SO_4$ | 1.4 | 95.3 | >95 | 4 |
| PentCs | 66.00 | Benzoic Acid | 61.00 | heptane | 0.12g $H_2SO_4$ | 1.2 | 96.2 | 78.0 | 4 |
| HxCb | 206.83 | Benzoic Acid | 132.66 | heptane | .37g $H_2SO_4$ | 3.9 | 89.8 | 97.5 | 4 |
| PM | 218.70 | Malonic Acid | 42.08 | none | 0.30g $H_2SO_4$ | 1.5 | 86.2 | 98.2 | 4 |
| DiEPh | 50.00 | Benzoyl Chloride | 38.58 | none | none | 0.3 | 95.5 | 99.1 | 2 |
| EPh | 30.00 | Benzoyl Chloride | 30.51 | none | none | 0.6 | 98.8 | n/a-solid | 2 |
| HxCb | 50.00 | Benzoyl Chloride | 36.93 | none | none | 1.6 | 95.8 | 98.1 | 2 |
| DiPPh | 40.00 | Benzoyl Chloride | 26.78 | none | none | 1.3 | 96.9 | 99.0 | 2 |

(continued)

| Glycol Ether | | Carboxylic acid or Ester Used | | Solvent used | Catalyst Used | Crude Rx Mixture Composition (Area %) | | Distilled Product | Preparation method |
|---|---|---|---|---|---|---|---|---|---|
| HTG | 40.00 | Benzoyl Chloride | 30.30 | none | none | 9.0 | 88.0 | 96.7 | 2 |
| TPPent | 40.00 | Benzoyl Chloride | 25.45 | none | none | 1.0 | 89.8 | 97.3 | 2 |
| DP2EH | 40.00 | Benzoyl Chloride | 25.45 | none | none | 0.0 | 91.7 | 94.8 | 2 |
| DPnB | 50.00 | Benzoyl Chloride | 36.36 | none | none | 4.3 | 92.7 | 98.6 | 2 |
| TPnB | 35.00 | Benzoyl Chloride | 19.39 | none | none | 4.7 | 90.2 | 93.8 | 2 |
| EPh | 44.60 | Succinyl Chloride | 24.77 | none | none | n/a-solid | n/a-solid | n/a-solid | 2 |
| HxCs | 44.00 | Succinyl Chloride | 23.39 | none | none | 1.7 | 91.7 | 96.5 | 2 |
| BuCb | 44.00 | Succinyl Chloride | 20.64 | none | none | 1.9 | 92.5 | 94.4 | 2 |
| TPM | 45.00 | Succinyl Chloride | 16.51 | none | none | 2.6 | 71.5 | 91.4 | 2 |
| DPnP | 40.00 | Succinyl Chloride | 17.20 | none | none | 8.6 | 84.1 | 95.3 | 2 |
| DPnB | 40.00 | Succinyl Chloride | 15.82 | none | none | 7.4 | 84.7 | 93.8 | 2 |
| BuCb | 44.00 | Adipoyl Chloride | 23.92 | none | none | 0.2 | 93.0 | 98.3 | 2 |
| DPM | 35.00 | Adipoyl Chloride | 21.34 | none | none | 0.0 | 93.1 | 94.5 | 2 |
| TPM | 35.00 | Adipoyl Chloride | 15.69 | none | none | 2.6 | 88.8 | 95.9 | 2 |

(continued)

| Glycol Ether | | Carboxylic acid or Ester Used | | Solvent used | Catalyst Used | Crude Rx Mixture Composition (Area %) | | Distilled Product | Preparation method |
|---|---|---|---|---|---|---|---|---|---|
| DPnB | 35.00 | Adipoyl Chloride | 16.32 | none | none | 0.1 | 98.4 | 98.6 | 2 |
| DPnP | 35.00 | Adipoyl Chloride | 18.18 | none | none | 1.1 | 97.5 | 98.5 | 2 |
| PnB | 35.00 | Adipoyl Chloride | 18.70 | none | none | 0.0 | 80.1 | 94.8 | 2 |
| TPM | 107.15 | Maleic Anhydride | 25.05 | heptane | 0.14g $H_2SO_4$ | 27.2 | 66.0 | 93.1 | 1 |
| BuCb | 85.51 | Maleic Anhydride | 25.07 | heptane | 0.16g $H_2SO_4$ | 10.5 | 85.0 | 96.8 | 1 |
| HxCs | 75.53 | Maleic Anhydride | 25.00 | heptane | 0.14g $H_2SO_4$ | 6.4 | 87.7 | 99.0 | 1 |
| DPnB | 106.72 | Maleic Anhydride | 25.11 | heptane | 0.14g $H_2SO_4$ | 16.3 | 82.8 | 99.2 | 1 |
| PM | 139.09 | Maleic Anhydride | 37.80 | heptane | 0.21g $H_2SO_4$ | 1.5 | 72.9 | 98.6 | 1 |

| Key to Glycol Ether abbreviations | | | | | |
|---|---|---|---|---|---|
| DiEPh | Diethylene glycol phenyl ether | TPnB | Tripropyl lene glycol n-butyl ether | BuCs | Ethylene glycol n-butyl ether |
| HxCb | Diethylene glycol nhexyl ether | BuCb | Diethylene glycol n-butyl ether | PentCs | Ethyllene glycol ol n-pentyl, ether |
| HxCs | Ethylene glycol n- hexyl ether | DPnP | Dipropylene glycol n-propyl ether | EPh | Ethylene glycol phenyl ether |
| DiPPh | Dipropylene glycol phenyl ether | DPM | Dipropylene glycol methyl ether | TPPent | Tripropylene glycol n-pentyl ether |
| TPM | Tripropylene glycol metry ether | PM | Propylene glycol methyl ether | DP2EH | Dipropylene glycol 2-ethylhexyl ether |

(continued)

| Key to Glycol Ether abbreviations | | | | | |
|---|---|---|---|---|---|
| TPnP | Tripropylene glycol n-propyl ether | DPnB | Dipropylene glycol n-butyl ether | PnB | Propylene glyco in-butyl ether |
| BTG | Triethylene glycol n- butyl ether | HTG | Triethylene glycol n-hexyl ether | | |

Table 1.2 Glycol ether-ester coalescents, their properties and MFFTs of aqueous polymeric dispersion compositions including them (Compounds 1 to 28, 32, 38, 40-42 are not of the present invention, but included for comparison purposes)

| | Coalescent Type None (neat polymeric dispersion) | Chemical Name none | Percent Volatility (EPA Method 24) n.a | Boiling Point (°C @ 760 mmHg) n.a | MFFT (°F) of RHOPLEX™ SG-30 with 5% coalescent based on polymer solids) >54 (neat emulsion polymer) |
|---|---|---|---|---|---|
| 1 | Ester alcohol | 2,2,4-trimethyl-1,3-pentanediol monoisobutyrate (Texanol®) | 99.8 | 255 | 44 |
| 2 | Glycol diester | Triethylene glycol bis-2-ethylhexanoate (Optitilm® Enhancer 400) | 1.1 | 422 | 39 |
| 3 | Bis-alkyl ester | Bis (2-ethyl hexyl) adipate | 0.8 | 417 | >50 |
| 4 | Glycol ether ester | Triethylene glycol n-hexyl ether benzoate | 0.4 | 441 | 41 |
| 5 | Glycol ether ester | Dipropylene glycol 2-ethylhexyl ether benzoate | 4.3 | 420 | 40 |
| 6 | Glycol ether ester | Diethylene glycol n-hexyl ether benzoate | 3.5 | 390 | 38 |
| 7 | Glycol ether ester | Ethylene glycol phenyl ether benzoate | 2.6 (solid) | 370 | not tested |
| 8 | Glycol ether ester | Diethylene glycol phenyl ether benzoate | 0.7 | 440 | 45 |
| 9 | Glycol ether ester | Tripropylene glycol n-pentyl ether benzoate | 2.2 | 425 | 40 |
| 10 | Glycol ether ester | Dipropylene glycol phenyl ether benzoate | 1.5 | 422 | 40 |
| 11 | Glycol ether ester | Dipropylene glycol n-butyl ether benzoate | 10.7 | 375 | 43 |
| 12 | Glycol ether ester | Tripropylene glycol n-butyl ether benzoate | 4.3 | 410 | 43 |
| 13 | Glycol ether ester | Ethylene glycol n-pentyl ether benzoate | 45.6 | 305 | 41 |
| 14 | Glycol ether ester | Ethylene glycol n-butyl ether benzoate | 78.1 | 290 | 41 |
| 15 | Glycol ether ester | Triethylene glycol n-pentyl ether benzoate | 1.7 | 425 | n.a |
| 16 | Glycol ether ester | Dipropylene glycol phenyl ether levulinate | 2.6 | 414 | 46 |
| 17 | Glycol ether ester | Ethylene glycol n-hexyl ether levulinate | 37.4 | 332 | 42 |

(continued)

| | Coalescent Type None (neat polymeric dispersion) | Chemical Name none | Percent Volatility (EPA Method 24) n.a | Boiling Point (°C @ 760 mmHg) n.a | MFFT (°F) of RHOPLEX™ SG-30 with 5% coalescent based on polymer solids) >54 (neat emulsion polymer) |
|---|---|---|---|---|---|
| 18 | Glycol ether ester | Diethylene glycol n-hexyl ether levulinate | 10.6 | 383 | 40 |
| 19 | Glycol ether ester | Diethylene glycol phenyl ether levulinate | 1.2 | 420 | 40 |
| 20 | Glycol ether ester | Tripropylene glycol methyl ether levulinate | 6.9 | 367 | not tested |
| 21 | Glycol ether ester | Tripropylene glycol n-propyl ethet levulinate | 7.2 | 385 | not tested |
| 22 | Glycol ether ester | Triethylene glycol n-butyl ether levulinate | 3.6 | 403 | 45 |
| 23 | Glycol ether ester | Tripropylene glycol n-butyl ether levulinate | 3.1 | 403 | 40 |
| 24 | Glycol ether ester | Tripropylene glycol n-butyl ether isopentanoate | 27.5 | 390 | 43 |
| 25 | Glycol ether ester | Diethylene glycol phenyl ether isopentanoate | 10 | 382 | 45 |
| 26 | Glycol ether ester | Triethylene glycol n-hexyl ether isopentanoate | 8.1 | 396 | 40 |
| 27 | Glycol ether ester | Triethylene glycol n-butyl ether isopentanoate | 20.6 | 360 | 41 |
| 28 | Glycol ether ester | Triethylene glycol n-hexyl ether valerate | 5.8 | 398 | 45 |
| 29 | Bis-Glycol ether ester | Bis-Ethylene glycol phenyl ether succinate | solid | 485 | not tested |
| 30 | Bis-glycol ether ester | Bis-Diethylene glycol n-butyl ether succinate | 0.4 | 452 | 40 |
| 31 | Bis-glycol ether ester | Bis-Propylene glycol phenyl ether succinate | 0.3 (solid) | 483 | not tested |
| 32 | Bis-glycol ether ester | Bis-Ethylene glycol n-hexyl ether succinate | 0.8 | 430 | 41 |
| 33 | Bis-glycol ether ester | Bis-Tripropylene glycol methyl ether succinate | 1.6 | 464 | 42 |
| 34 | Bis-glycol ether ester | Bis-Dipropylene glycol n-propyl ether succinate | 1.8 | 450 | 41 |
| 35 | Bis-glycol ether ester | Bis-Dipropylene glycol n-butyl ether succinate | 1.0 | 460.0 | 41 |

(continued)

| | Coalescent Type None (neat polymeric dispersion) | Chemical Name none | Percent Volatility (EPA Method 24) n.a | Boiling Point (°C @ 760 mmHg) n.a | MFFT (°F) of RHOPLEX™ SG-30 with 5% coalescent based on polymer solids) >54 (neat emulsion polymer) |
|---|---|---|---|---|---|
| 36 | Bis-glycol ether ester | Bis-Diethylene glycol n-butyl ether maleate | 0.5 | 476.0 | 43 |
| 37 | Bis-glycol ether ester | Bis-Ethylene glycol n-hexyl ether maleate | 0.8 | 456.0 | 43 |
| 38 | Bis-glycol ether ester | Bis-Tripropylene glycol methyl ether maleate | 4.3 | 449.0 | 46 |
| 39 | Bis-glycol ether ester | Bis-Dipropylene glycol n-butyl ether maleate | 0.1 | 476.0 | 41 |
| 40 | Bis-glycol ether ester | Bis-Propylene glycol methyl ether maleate | 33.1 | 380.0 | 47 |
| 41 | Bis-glycol ether ester | Bis-Diethylene glycol n-hexyl ether malonate | 1.7 | 440 | not tested |
| 42 | Bis-glycol ether ester | Bis-Propylene glycol methyl ether malonate | 72.5 | 330 | 48 |
| 43 | Bis-glycol ether ester | Bis-Diethylene glycol n-butyl ether adipate | 0.5 | 479 | 40 |
| 44 | Bis-glycol ether ester | Bis-Tripropylene glycol methyl ether adipate | 0.9 | 471 | 41 |
| 45 | Bis-glycol ether ester | Bis-Dipropylene glycol n-butyl ether adipate | 0.2 | 485 | 40 |
| 46 | Bis-glycol ether ester | Bis-Dipropylene glycol n-propyl ether adipate | 0.5 | 470 | 41 |

EXAMPLE 5 - Evaluation of Diethylene Glycol Phenyl Ether Benzoate and Dipropylene Glycol Phenyl Ether Benzoate as Coalescents for an aqueous Epoxy Dispersion.

Glycol ether benzoates of the invention were added to an aqueous dispersion of a solid epoxy having a particle size of approximately 500nm. The aqueous polymeric dispersion is part of a 2k system, typically combined with amine-based curing agents for ambicure coatings at concentrations. The coalescecents were added at 4% by weight based on resin solids and the MFFT values compared with those obtained with no coalescent and with commercially available coalescents such as DOWANOL™ PPh. The MFFT of the coating compositions containing the glycol ether benzoates of the invention (Coating Compositions 1-2) were comparable to the MFFT obtained with DOWANOL™ PPh (about 6 °C) and considerably lower than the MFFT obtained without coalescing aids (about 12 °C). Aqueous gloss enamel coating compositions were prepared with either the glycol ether benzoates or the comparative coalescents at the 4% level based on resin solids. See Table 5.1 below. There was no significant loss of pot life in the presence of the benzoates. Evaluation of drawdowns of the cured formulations showed no detrimental plasticizing effects, no loss of gloss as a function of pot life, and no loss in water or chemical resistance in the coatings prepared with the benzoates (Tables 5.2 and 5.3). Hardness and flexibility also paralleled those obtained with the comparative formulations. (Table 5.4)

Table 5.1 - Aqueous Gloss Enamel Epoxy Coating Composition

| | | Coating Composition Comparative A | Coating Composition Comparative B | Coating Composition 1 | Coating Composition 2 |
|---|---|---|---|---|---|
| Ingredients (in lbs) | | | | | |
| Part A Grind | | | | | |
| Epoxy | | 200.00 | 200.00 | 200.00 | 200.00 |
| Water | | 29.15 | 29.15 | 19.75 | 19.75 |
| Sodium Nitrite (15%) | | 9.00 | 9.00 | 9.00 | 9.00 |
| Disperbyk™ 194 | | 29.40 | 29.40 | 29.40 | 29.40 |
| BYK-019 | | 2.00 | 2.00 | 2.00 | 2.00 |
| Ti-Pure™ R-706 TiO2 | | 240.40 | 240.40 | 240.40 | 240.40 |
| | | | | | |
| Part A Let Down | | | | | |
| Epoxy | | 334.34 | 334.34 | 334.34 | 334.34 |
| Di PPh Benzoate | | 0.00 | 0.00 | 10.00 | 0.00 |
| DiEPh Benzoate | | 0.00 | 0.00 | 0.00 | 10.00 |
| Tego Airex™ 902W | | 4.40 | 4.40 | 4.40 | 4.40 |
| Part B | | | | | |
| AP Anquamine™ 401 | | 100.44 | 100.44 | 100.44 | 100.44 |
| Arcosoly™ PM | | 8.90 | 0.00 | 0.00 | 0.00 |
| Dowanol™ PPh | | 8.90 | 0.00 | 0.00 | 0.00 |
| Dowanol™ PnP | | 0.00 | 17.90 | 0.00 | 0.00 |
| Water | | 98.61 | 96.40 | 116.65 | 116.65 |
| Properties | Units | | | | |
| Total Volume | gal | 100.00 | 100.00 | 100.00 | 100.00 |
| Total Weight | lbs | 1065.54 | 1063.43 | 1066.38 | 1066.38 |
| Total PVC wo Add | Percent | 18.00% | 18.00% | 18.00% | 18.00% |
| Volume Solids wo Add | Percent | 40.00% | 40.00% | 40.00% | 40.00% |
| Weight Solids wo Add | Percent | 52.73% | 52.83% | 52.69% | 52.69% |

(continued)

| Properties | Units | | | | |
|---|---|---|---|---|---|
| VOC | g/l | 50 | 50 | 2 | 2 |

Table 5.2 - Drying Test Results for Epoxy Coating Composition

| | Coating Composition Comparative A | Coating Composition Comparative B | Coating Composition 1 | Coating Composition 2 |
|---|---|---|---|---|
| Dry-to-Touch | 60 | 60 | 90 | 60 |
| Dry-to-Handle | 300 | 270 | 300 | 330 |
| Early Water Resistance (on Al) | | | | |
| 4hr dry, Blister Rating | 10 | 10 | 10 | 10 |
| 6hr dry, Blister Rating | 10 | 10 | 10 | 10 |

Table 5.3 - Resistance to Chemical attack of Epoxy Coating

| | Coating Comparative B | | Coating Comparative A | | Coating 1 | | Coating 2 | |
|---|---|---|---|---|---|---|---|---|
| Chemical Resistance | 24 hour Spot Test | 48 hour Spot Test | 24 hour Spot Test | 48 hour Spot Test | 24 hour Spot Test | 48 hour Spot Test | 24 hour Spot Test | 48 hour Spot Test |
| *Rating scale 1-5, 5=no film damage, 1=film dissolved, delaminated* | | | | | | | | |
| 10%H2 SO4 | 2 | 1.5 | 2 | 1.5 | 2 | 1.5 | 2 | 1.5 |
| 10%HCl | 3 | 1.5 | 3 | 1.5 | 3 | 1.5 | 3 | 1.5 |
| 30%am monia | 4.5 | 5 | 4.5 | 5 | 4.5 | 5 | 4.5 | 5 |
| 15%Na OH | 5 | 4 | 5 | 4.5 | 5 | 4 | 5 | 4 |
| MEK | 4 | 3.5 | 4 | 3.5 | 4 | 3.5 | 4 | 4 |
| gasoline | 4 | 4 | 4.5 | 4 | 4 | 4 | 4 | 4 |
| brake fluid | 3 | 3.5 | 3 | 3.5 | 3 | 3.5 | 3 | 3.5 |
| water | 4.5 | 5 | 4.5 | 5 | 4.5 | 5 | 4.5 | 5 |
| transmi ssion fluid | 5 | 4 | 5 | 4 | 5 | 4 | 5 | 4 |
| WD-40 | 5 | 5 | 5 | 5 | 5 | 5 | 5 | 5 |

(continued)

| | Coating Comparative B | | Coating Comparative A | | Coating 1 | | Coating 2 | |
|---|---|---|---|---|---|---|---|---|
| Chemical Resistance | 24 hour Spot Test | 48 hour Spot Test | 24 hour Spot Test | 48 hour Spot Test | 24 hour Spot Test | 48 hour Spot Test | 24 hour Spot Test | 48 hour Spot Test |
| *Rating scale 1-5, 5=no film damage, 1=film dissolved, delaminated* | | | | | | | | |
| Motor Oil | 5 | 4 | 5 | 4 | 5 | 4 | 5 | 4 |
| Coffee | 3.5 | 4 | 3.5 | 4 | 3.5 | 4 | 3.5 | 4 |
| Mustard | 2.5 | 2 | 2.5 | 2 | 2.5 | 2.5 | 2.5 | 2.5 |
| 50% Ethanol | 4.5 | 4 | 4.5 | 4 | 4.5 | 4 | 4.5 | 4 |
| Skydrol TM | 5 | 4 | 5 | 4 | 4.5 | 4 | 5 | 4 |
| IPA | 5 | 4 | 5 | 4 | 5 | 4.5 | 5 | 4.5 |

Table 5.4 - Hardness/Flexibility Performance of Epoxy Coating

| | Coating Composition Comparative B | Coating Composition Comparative A | Coating Composition 1 | Coating Composition 2 |
|---|---|---|---|---|
| Konig Hardness (seconds) | | | | |
| 1day | 104 | 83 | 83 | 79 |
| 7day | 136 | 117 | 120 | 118 |
| 14day | 134 | 117 | 121 | 118 |
| 30day | 131 | 118 | 127 | 124 |
| Pencil Hardness | | | | |
| 1day | 2H | 2H | 2H | H |
| 7day | 4H | 5H | 5H | 3H |
| 14day | 6H | 6H | 5H | 6H |
| 30day | 6H | 6H | 7H | 6H |
| Impact resistance | | | | |
| direct, in lb | 10 | 20 | 20 | 30 |
| inverse, in lb | <4 | <4 | <4 | <4 |
| Mandrel Flex | | | | |
| Rod Diameter | | | | |
| 1/2" | F | P | P | P |
| 1/4" | F | P | P | P |
| 1/8" | F | F | F | F |

EXAMPLE 6 - Performance of Glycol Ether Esters and Diesters in an aqueous coating composition including an emulsion polymer

A master aqueous coating batch was prepared having the composition in Table 6.1 and all test coalescents were post added at 8% by weight based on resin solids. A total of 14 formulations were evaluated including controls with TEXANOL™, DOWANOL™ DPnB, and OPTIFILM™ 400. A series of typical paint tests were conducted on drawdowns of each formulation. These tests were gloss, low temperature film formation (LTFF), yellowing, 1-day hot block, 1-day oven print, Konig hardness, 1-day dry-wet alkyd adhesion, scrub, dirt pick up resistance (DPUR) and color acceptance. The results of these tests showed that the glycol ether esters and diesters of the invention performed well in a fully formulated coating composition(Tables 6.2 and 6.3).

Table 6.1 - RHOPLEX™ SG-10M acrylic emulsion polymer Semi-Gloss Formulation

| Component | Pounds | Gallons | |
|---|---|---|---|
| Grind | | | |
| TI-PURE™ R-746 | 341.3 | 17.56 | |
| water | 30 | 3.59 | |
| Propylene glycol | 28 | 3.24 | |
| TAMOL™ 165A | 8.7 | 0.98 | |
| TRITON™ GR-7M | 2.1 | 0.24 | |
| KATHON™ LX 1.5% | 1.8 | 0.21 | |
| Grind sub-total | 411.9 | 25.82 | |
| LetDown | | | |
| FOAMASTER™ VL | 2 | 0.25 | |
| RHOPLEX™ SG-10M | 494.79 | 56.01 | |
| Water | 104.36 | 12.5 | |
| ACRYSOL™ RM 2020 NPR | 20 | 2.3 | |
| ACRYSOL™ RM-8W | 5.3 | 0.61 | |
| Coalescent | 19.79 | 2.1 - 2.6 | (8% on polymer solids) |
| Totals --> | 1058.14 | 99.59 - 100.09 | (volume a factor of coalescing solvent density) |
| Weight solids --> | 48.05% | | 23% PVC |

Table 6.2 - Results of Tests with Aqueous coating composition

| | LTFF (40/40) | 1 day Gloss | Brush Flow | Contrast Ratio | Color Acceptance | Yellowing - HA film |
|---|---|---|---|---|---|---|
| Texanol™ | 99/97 | 34/73 | 8 | .9518 | Good | Excellent |
| Dowanol™ DPnB | 99/99 | 26/65 | 7+ | .9259 | Light | Excellent |
| Optitilm™ 400 | 99/99 | 37/75 | 7+ | .9432 | S1. Light | Excellent |
| Butyl Carbitol™ Adipate | 99/97 | 37/75 | 7 | .9353 | S1. Light | Excellent |
| DPnB Adipate | 99/97 | 32/75 | 7 | .9465 | S1. Light | Excellent |
| DPnP Adipate | 99/99 | 36/74 | 7+ | .9454 | S1. Light | Excellent |
| Butyl Carbitol™ Succinate | 99/99 | 37/75 | 6 | .9363 | S1. Light | Excellent |
| Hexyl Cellosolve Maleate | 99/99 | 38/75 | 7 | .9453 | S1. Light | Excellent |
| Butyl Carbitol Maleate | 99/97 | 38/76 | 6+ | .9432 | S1. Light | Excellent |
| Hexyl Carbitol Benzoate | 99/99 | 35/73 | 7+ | .9408 | S1. Light | Excellent |
| DPnB Maleate | 99/97 | 37/75 | 7 | .9432 | S1. Light | Excellent |

(continued)

| | LTFF (40/40) | 1 day Gloss | Brush Flow | Contrast Ratio | Color Acceptance | Yellowing - HA film |
|---|---|---|---|---|---|---|
| DiEPh Technical Benzoate | 99/97 | 38/76 | 6+ | .9463 | S1. Light | Excellent |
| TPP Benzoate | 99/99 | 38/75 | 6+ | .9400 | S1. Light | Excellent |
| PTG Benzoate | 99/99 | 38/76 | 6 | .9453 | S1. Light | Excellent |

Table 6.3 - Results of Tests with Coatings

| | Abrasive Scrub | Stain Removal Hydrophobic/Hydrophilic. | 1 day Hot Block | 1 day Oven Print | Konig Hardness (1/7/14 days) | Lab DPUR % retained |
|---|---|---|---|---|---|---|
| Texanol™ | 1073 | 77/53 | 8+ | 8+ | 13/20 | 99.8 |
| Dowanol™ DPnB | 793 | 65/53 | 8+ | 9+ | 15/32 | 99.6 |
| Optifilm™ 400 | 1127 | 71/53 | 8+ | 6+ | 9/11 | 98.5 |
| Butyl Carbitol™ Adipate | 1065 | 57/73 | 8+ | 6 | 9/11 | 98.9 |
| DPnB Adipate | 1178 | 56/63 | 8+ | 6 | 10/13 | 97.2 |
| DPnP Adipate | 1059 | 57/53 | 8+ | 6 | 10/12 | 98.6 |
| Butyl Carbitol™ Succinate | 1222 | 56/53 | 8+ | 6 | 9/11 | 98.4 |
| Hexyl Cellosolve™ | 1040 | 68/53 | 8+ | 6 | 9/11 | 97.6 |
| Maleate | | | | | | |
| Butyl Carbitol™ Maleate | 1172 | 69/53 | 8+ | 7 | 10/12 | 99.2 |
| Hexyl Carbitol Benzoate | 1072 | 63/53 | 8+ | 6 | 9/11 | 98.3 |
| DPnB Maleate | 1027 | 71/53 | 8+ | 7 | 11/13 | 99.3 |
| DiEPh Technical Benzoate | 872 | 73/53 | 8+ | 5+ | 12/14 | 98.1 |
| TPP Benzoate | 1309 | 68/53 | 8+ | 6 | 10/13 | 98.2 |
| PTG Benzoate | 1154 | 68/53 | 8+ | 6 | 9/11 | 99.4 |

EXAMPLE 7. Performance of Glycol Ether Esters and Diesters with Opaque Polymers

Certain glycol ether-esters and diesters were compared with TEXANOL™ and OPTIFILM™ 400 as coalescents in their ability to preserve the opacity provided by several commercial ROPAQUE™ opaque polymers (multistage emulsion polymers including, when dry, a void) in a standard test formulation. All coalescing aids were evaluated at 10% by weight based on resin solids. In Table 7.1, high values stand for high scattering and preservation of opacity. It can be

seen that glycol ether esters and diesters like dipropylene glycol phenyl ether benzoate (DiPPh Benzoate), bis-dipropylene glycol n-butyl ether adipate (DPnB Adipate), bis-dipropylene glycol n-propyl ether adipate (DPnP Adipate), bis-dipropylene glycol n-butyl ether maleate (DPnB Maleate), and tripropylene glycol pentyl ether benzoate (TPP Benzoate) had similar performance to TEXANOL™with ROPAQUE™ Dual and better performance with ROPAQUE™ Dual, ROPAQUE™ Ultra, and/or ROPAQUE™ Ultra E than OPTIFILM™ 400.

Table 7.1 Performance with opaque polymers

| Coalescent | Opaque Polymer - Relative Scattering | | |
|---|---|---|---|
| | ROP AQUE™ ULTRA E | ROP AQUE™ ULTRA | ROP AQUE™ DUAL |
| TEXANOL™ | 100 | 100 | 100 |
| DiPPh Benzoate | NA | 81 | 102 |
| DPnB Adipate | 7 | NA | 102 |
| DPnP Adipate | 26 | NA | 99 |
| DPnB Maleate | 69 | NA | 98 |
| TPGPE Benzoate | 8 | 59 | 97 |
| OPTIFILM™400 | 3 | 19 | 84 |
| Hexyl CELLOSOL VE™ Maleate | 13 | NA | 76 |
| Butyl CARBITOL™ Maleate | 36 | NA | 75 |

Certain glycol ether esters and diesters such as dipropylene glycol phenyl ether benzoate (DiPPh Benzoate), bis-dipropylene glycol n-butyl ether adipate (DPnB Adipate), bis-dipropylene glycol n-propyl ether adipate (DPnP Adipate), bis-dipropylene glycol n-butyl ether maleate (DPnB Maleate), and tripropylene glycol pentyl ether benzoate (TPP Benzoate) of the invention had similar performance to TEXANOL™with ROPAQUE™ Dual and better performance with ROPAQUE™ Dual, ROPAQUE™ Ultra, and/or ROPAQUE™ Ultra E than OPTIFILM™ 400.

## Claims

1. An aqueous coating composition comprising an aqueous polymeric dispersion and from 0.1% to 40% by weight, based on the weight of said aqueous polymeric dispersion solids of a glycol ether-ester coalescent selected from the group of compositions of Formula (II):

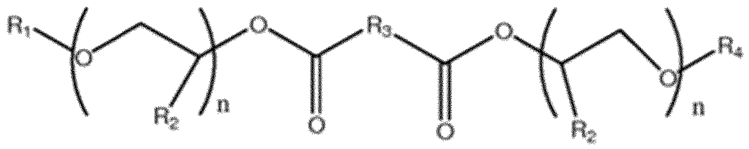

(II)

wherein $R_1$ and $R_4$ are, independently, $C_1$-$C_{10}$ alkyl groups, phenyl or benzyl, $R_2$ is either hydrogen or methyl, $R_3$ is a carbon chain comprising 3-4 carbon atoms, and n = 1-4; and mixtures thereof; wherein the boiling point of said coalescent is greater than 450 °C at 760 mm Hg measured as indicated in the description.

2. The aqueous coating composition of claim 1 wherein said coalescent is bis-dipropylene glycol n-butyl ether adipate.

3. The aqueous coating composition of claim 1 or 2 wherein said aqueous polymeric dispersion comprises an epoxy emulsion.

4. The aqueous coating composition of any of claims 1 to 3 wherein said aqueous polymeric dispersion has a minimum film formation temperature (MFFT) of from -5 °C to 100 °C, said coating composition comprising from 0.1% to 30% by weight, based on the weight of said aqueous polymeric dispersion solids, said coalescent composition of claim 1, wherein a MFFT of the aqueous polymeric dispersion are measured using ASTM D2354 and a 5 mil MFFT bar.

5. The aqueous coating composition of any of claims 1 to 4 further comprising a multistage emulsion polymer that, when dry, includes a void.

6. A method for forming a coating comprising:

(a) forming said aqueous coating composition of any of claims 1 to 5;
(b) applying said aqueous coating composition to a substrate; and
(c) drying, or allowing to dry, said applied aqueous coating composition.

**Patentansprüche**

1. Eine wässrige Beschichtungszusammensetzung, beinhaltend eine wässrige polymere Dispersion und bezogen auf das Gewicht der Feststoffe der wässrigen polymeren Dispersion zu 0,1 Gew.-% bis 40 Gew.-% ein Glykoletheresterkoaleszenzmittel, das aus der Gruppe von Zusammensetzungen der Formel (II) ausgewählt ist:

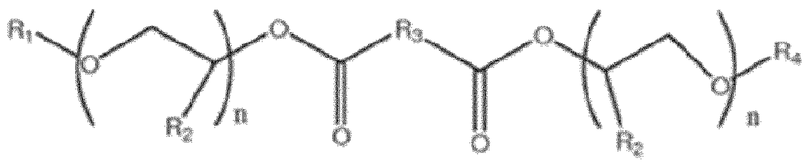
(II)

wobei $R_1$ und $R_4$ unabhängig $C_1$-$C_{10}$-Alkylgruppen, Phenyl oder Benzyl sind, $R_2$ entweder Wasserstoff oder Methyl ist, $R_3$ eine Kohlenstoffkette, die 3-4 Kohlenstoffatome beinhaltet, ist und n = 1-4; und Mischungen davon; wobei der Siedepunkt des Koaleszenzmittels bei 760 mmHg höher als 450°C ist, gemessen wie in der Beschreibung angegeben.

2. Wässrige Beschichtungszusammensetzung gemäß Anspruch 1, wobei das Koaleszenzmittel Bis-dipropylenglykol-n-butyletheradipat ist.

3. Wässrige Beschichtungszusammensetzung gemäß Anspruch 1 oder 2, wobei die wässrige polymere Dispersion eine Epoxidemulsion beinhaltet.

4. Wässrige Beschichtungszusammensetzung gemäß einem der Ansprüche 1 bis 3, wobei die wässrige polymere Dispersion eine Mindestfilmbildetemperatur (MFFT) von -5°C bis 100°C aufweist, wobei die Beschichtungszusammensetzung bezogen auf das Gewicht der Feststoffe der wässrigen polymeren Dispersion zu 0,1 Gew.-% bis 30 Gew.-% die Koaleszenzmittelzusammensetzung gemäß Anspruch 1 beinhaltet, wobei eine MFFT der wässrigen polymeren Dispersion unter Verwendung von ASTM D2354 und einer 5-mil-MFFT-Stange gemessen wird.

5. Wässrige Beschichtungszusammensetzung gemäß einem der Ansprüche 1 bis 4, die ferner ein mehrstufiges Emulsionspolymer beinhaltet, das im trockenen Zustand einen Hohlraum umfasst.

6. Ein Verfahren zum Bilden einer Beschichtung, das Folgendes beinhaltet:

(a) Bilden der wässrigen Beschichtungszusammensetzung gemäß einem der Ansprüche 1 bis 5;
(b) Auftragen der wässrigen Beschichtungszusammensetzung auf ein Substrat; und
(c) Trocknen oder Trocknenlassen der aufgetragenen wässrigen Beschichtungszusammensetzung.

**Revendications**

1. Une composition de revêtement aqueuse comprenant une dispersion polymérique aqueuse et de 0,1 % à 40 % en poids, rapporté au poids desdits solides de la dispersion polymérique aqueuse d'un coalescent ester-éther de glycol sélectionné dans le groupe de compositions de Formule (II) :

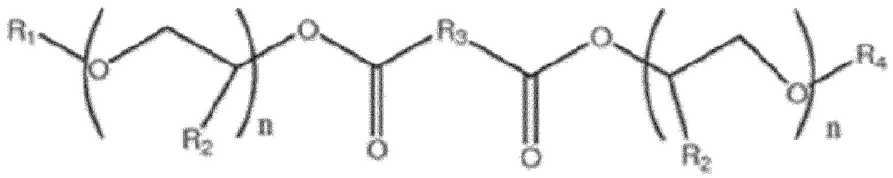

(II)

dans laquelle $R_1$ et $R_4$ sont, indépendamment, des groupes alkyle en $C_1$-$C_{10}$, un phényle ou un benzyle, $R_2$ est soit l'hydrogène, soit un méthyle, $R_3$ est une chaîne carbonée comprenant 3 à 4 atomes de carbone, et n = 1 à 4 ; et des mélanges de celles-ci ; dans laquelle le point d'ébullition dudit coalescent est supérieur à 450 °C à 760 mm Hg mesuré tel qu'indiqué dans la description.

**2.** La composition de revêtement aqueuse de la revendication 1 dans laquelle ledit coalescent est un adipate de bis-éther n-butylique de dipropylène glycol.

**3.** La composition de revêtement aqueuse de la revendication 1 ou de la revendication 2 dans laquelle ladite dispersion polymérique aqueuse comprend une émulsion d'époxy.

**4.** La composition de revêtement aqueuse de n'importe lesquelles des revendications 1 à 3 dans laquelle ladite dispersion polymérique aqueuse a une température minimale de formation de film (TMFF) allant de -5 °C à 100 °C, ladite composition de revêtement comprenant de 0,1 % à 30 % en poids, rapporté au poids desdits solides de la dispersion polymérique aqueuse, de ladite composition de coalescent de la revendication 1, dans laquelle une TMFF de la dispersion polymérique aqueuse se mesure en utilisant l'ASTM D2354 et un appareil MFFT bar 5 mil.

**5.** La composition de revêtement aqueuse de n'importe lesquelles des revendications 1 à 4 comprenant en sus un polymère en émulsion multiphases qui, lorsqu'il est sec, inclut un vide.

**6.** Un procédé pour former un revêtement comprenant le fait :

(a) de former ladite composition de revêtement aqueuse de n'importe lesquelles des revendications 1 à 5 ;
(b) d'appliquer ladite composition de revêtement aqueuse sur un substrat ; et
(c) de sécher, ou de laisser sécher, ladite composition de revêtement aqueuse appliquée.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 4489188 A
- US 20090198002 A1
- US 4384056 A
- US 4539361 A